# EUROPEAN PATENT APPLICATION

(11) **EP 4 749 488 A1**
(43) Date of publication of application: **27.05.2026**
(21) Application number: 23945876.3
(22) Date of filing: 19.07.2023
(51) Int. Cl.: G06F 21/32

(54) **INFORMATION PROCESSING SYSTEM, INFORMATION PROCESSING METHOD, AND RECORDING MEDIUM**

(71) Applicant: NEC Corporation, 108-8001 Tokyo (JP)
(72) Inventor: HAMADA, Hirokazu, Tokyo 108-8001 (JP)
(74) Representative: Betten & Resch
(86) International application number: PCT/JP2023/026434
(87) International publication number: WO 2025/017873

(57) **Abstract**

An information processing system includes: a first terminal that acquires a feature quantity of an echo sound reflected in a target's ear; a second terminal that enables a predetermined operation based on ear acoustic authentication using the feature quantity; a detection unit that detects contact between the first terminal and the second terminal; and a transmission unit that transmits the feature quantity from the first terminal to the second terminal upon detection of the contact. According to such an information processing system, it is possible to appropriately transmit the feature quantity used for the ear acoustic authentication from an acquisition source.

## Description

### Technical Field

The present disclosure relates to technical fields of an information processing system, an information processing method, and a recording medium.

### Background Art

There is known a technology/technique for performing target authentication processing (so-called ear acoustic authentication), based on characteristics of an external auditory canal acquired using sound reflections. For example, Patent Literature 1 discloses a technology/technique in which ear acoustic authentication is performed using an ear canal echo sound acquired at a first device, and in which the use of a second device is permitted in response to a success in the ear acoustic authentication.

### Citation List

### Patent Literature

[Patent Literature 1] JP2019-113947A

### Summary

### Technical Problem

It is an example object of the present disclosure to provide an information processing system, an information processing method, and a recording medium that are allowed to appropriately transmit a feature quantity used for ear acoustic authentication from an acquisition source.

### Solution to Problem

An information processing system according to an example aspect of the present disclosure includes: a first terminal that acquires a feature quantity of an echo sound reflected in a target's ear; a second terminal that enables a predetermined operation based on ear acoustic authentication using the feature quantity; a detection unit that detects contact between the first terminal and the second terminal; and a transmission unit that transmits the feature quantity from the first terminal to the second terminal upon detection of the contact.

An information processing method according to an example aspect of the present disclosure is an information processing method that is executed by at least one computer and that controls an information processing system including: a first terminal that acquires a feature quantity of an echo sound reflected in a target's ear; and a second terminal that enables a predetermined operation based on ear acoustic authentication using the feature quantity, the information processing method including: detecting contact between the first terminal and the second terminal; and transmitting the feature quantity from the first terminal to the second terminal upon detection of the contact.

A recording medium according to an example aspect of the present disclosure is a recording medium on which a computer program that allows at least one computer to execute an information processing method is recorded, the information processing method being executed by at least one computer and controlling an information processing system including: a first terminal that acquires a feature quantity of an echo sound reflected in a target's ear; and a second terminal that enables a predetermined operation based on ear acoustic authentication using the feature quantity, the information processing method comprising: detecting contact between the first terminal and the second terminal; and transmitting the feature quantity from the first terminal to the second terminal upon detection of the contact.

### Brief Description of Drawings

[FIG. 1] FIG. 1 is a block diagram illustrating a hardware configuration of a first information processing system.
[FIG. 2] FIG. 2 is a block diagram illustrating a terminal configuration of the first information processing system.
[FIG. 3] FIG. 3 is a flowchart illustrating a flow of operation of the first information processing system.
[FIG. 4] FIG. 4 is a block diagram illustrating a terminal configuration of a second information processing system.
[FIG. 5] FIG. 5 is a flowchart illustrating a flow of operation of the second information processing system.

### Description of Example Embodiments

Hereinafter, an information processing system, an information processing method, and a recording medium according to example embodiments will be described with reference to the drawings.

### <First Example Embodiment>

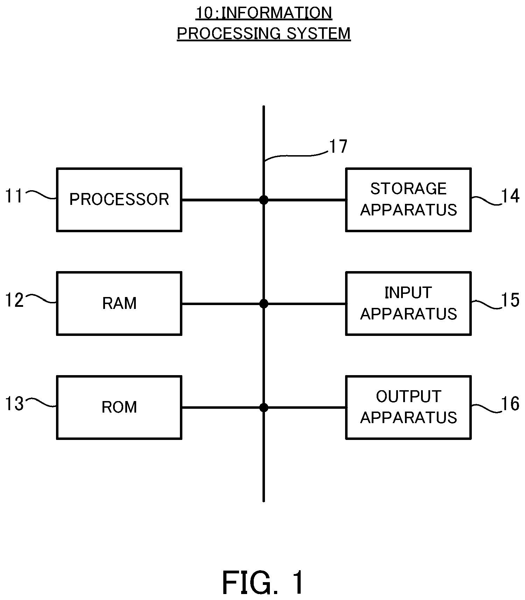

A first information processing system will be described with reference to FIG. 1 to FIG. 3.

### (Hardware Configuration)

First, with reference to FIG. 1, a hardware configuration of the first information processing system will be described. FIG. 1 is a block diagram illustrating the hardware configuration of the first information processing system.

As illustrated in FIG. 1, a first information processing system 10 includes a processor 11, a RAM (Random Access Memory) 12, a ROM (Read-Only Memory) 13, and a storage apparatus 14. The information processing system 10 may further include an input apparatus 15 and an output apparatus 16. The processor 11, the RAM 12, the ROM 13, the storage apparatus 14, the input apparatus 15, and the output apparatus 16 are connected via a data bus 17. The data bus 17 may be an interface other than a data bus (e.g., LAN, USB, etc.).

The processor 11 reads a computer program. For example, the processor 11 is configured to read a computer program stored in at least one of the RAM 12, the ROM 13, and the storage apparatus 14. Alternatively, the processor 11 may read a computer program stored on a computer-readable recording medium, by using a not-illustrated recording medium reading apparatus. The processor 11 may also acquire (i.e., read) a computer program from a not-illustrated apparatus disposed outside the information processing system 10 via a network interface. The processor 11 controls the RAM 12, the storage apparatus 14, the input apparatus 15, and the output apparatus 16 by executing the read computer program. Especially in the present example embodiment, when the processor 11 executes the read computer program, a function block for transmitting a feature quantity used for ear acoustic authentication is realized in the processor 11. That is, the processor 11 may function as a controller that performs each control in the information processing system 10.

The processor 11 may be configured, for example, as a CPU (Central Processing Unit), a GPU (Graphics Processing Unit), a FPGA (field-programmable gate array), a DSP (Digital Signal Processor), an ASIC (Application Specific Integrated Circuit), or a quantum processor. The processor 11 may be configured by using one of them, or a plurality of them in parallel.

The RAM 12 temporarily stores the computer program to be executed by the processor 11. The RAM 12 temporarily stores data that are temporarily used by the processor 11 when the processor 11 is executing the computer program. The RAM 12 may be, for example, a D-RAM (Dynamic Random Access Memory) or a SRAM (Static Random Access Memory). In addition, another type of volatile memory may be used in place of the RAM 12.

The ROM 13 stores the computer program to be executed by the processor 11. The ROM 13 may also store other fixed data. The ROM 13 may be, for example, a P-ROM (Programmable Read Only Memory) or an EPROM (Erasable Read Only Memory). In addition, another type of nonvolatile memory may be used in place of the ROM 13.

The storage apparatus 14 stores data that are stored by the information processing system 10 for a long time. The storage apparatus 14 may operate as a transitory storage apparatus of the processor 11. The storage apparatus may store therein the computer program to be executed by the processor 11. The storage apparatus 14 may include, for example, at least one of a hard disk apparatus, a magneto-optical disk apparatus, a SSD (Solid State Drive), and a disk array apparatus.

The input apparatus 15 is an apparatus that receives an input instruction from a user of the information processing system 10. The input apparatus 15 may include, for example, at least one of a keyboard, a mouse, and a touch panel. The input apparatus 15 may be configured as a part of a smartphone, a tablet terminal, an earphone-type terminal, a watch-type terminal, or the like. The input apparatus 15 may be an apparatus that allows audio input/voice input, including a microphone, for example. The input apparatus 15 may be used solely for maintenance purposes. In such a case, the input apparatus 15 may be omitted as appropriate from the components used when the information processing system 10 is operated.

The output apparatus 16 is an apparatus that outputs information about the information processing system 10 to the outside. For example, the output apparatus 16 may be a display apparatus (e.g., a display) that is configured to display the information about the information processing system 10. The output apparatus 16 may also be a speaker or the like that is configured to audio-output the information about the information processing system 10. The output apparatus 16 may be configured as a part of a smartphone, a tablet terminal, an earphone-type terminal, a watch-type terminal, or the like.

The first information processing system 10 is configured to include a first terminal and a second terminal, as described later. Each of the components described in FIG. 1 may be configured to be included in at least one of the first terminal and the second terminal provided in the first information processing system 10. Furthermore, a part of the components described in FIG. 1 may be configured to be included in an external apparatus (e.g., an external server or cloud) that is different from the first terminal and the second terminal.

### (Terminal Configuration)

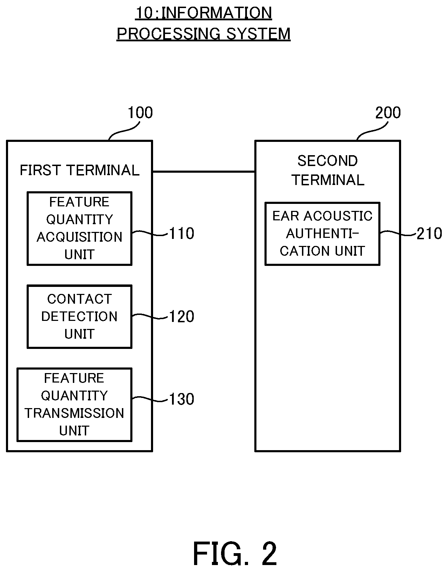

Next, with reference to FIG. 2, a terminal configuration of the first information processing system 10 will be described. FIG. 2 is a block diagram illustrating the terminal configuration of the first information processing system.

In FIG. 2, the first information processing system 10 includes a first terminal 100 and a second terminal 200. The first terminal 100 and the second terminal 200 are connected, for example, via wireless communication, and are configured to transmit and receive information with each other. The first terminal 100 is configured, for example, as an earphone-type hearable terminal, and includes, as processing blocks for realizing its functions: a feature quantity acquisition unit 110, a contact detection unit 120, and a feature quantity transmission unit 130. The second terminal 200 is configured as a portable terminal such as, for example, a smartphone, a tablet terminal, and a watch-type terminal, and includes an ear acoustic authentication unit 210 as a processing block for realizing its functions. Each of the feature quantity acquisition unit 110, the contact detection unit 120, the feature quantity transmission unit 130, and the ear acoustic authentication unit 210 may be a processing block configured, for example, by the aforementioned processor 11 (see FIG. 1).

The feature quantity acquisition unit 110 is configured to acquire a feature quantity of an echo sound reflected in a target's ear. The feature quantity acquisition unit 110, for example, controls the first terminal 100 worn on the target's ear to acquire an echo signal including characteristics/features of the target's ear (specifically, the characteristics/features of an inner shape of the ear). More specifically, the ear acoustic authentication unit 110 controls the first terminal 100 to send a sound wave into the target's ear, and acquires an echo signal based on an echo sound of the sent sound wave. The ear acoustic authentication unit 110 then extracts the feature quantity from the acquired echo signal. The feature quantity is a parameter indicating the characteristics/features of the inner shape of the target's ear, and is used for ear acoustic authentication in the ear acoustic authentication unit 210.

The feature quantity acquisition unit 110 may acquire the feature quantity, for example, in timing in which the target puts on the first terminal 100. Furthermore, the feature quantity acquisition unit 110 may periodically acquire the feature quantity at predetermined intervals, after the target puts on the first terminal 100. The feature quantity acquisition unit 110 may also acquire the feature quantity, in timing in which the target performs an operation for acquiring the feature quantity. The feature quantity acquisition unit 110 may also have a function for temporarily storing the acquired feature quantity.

The contact detection unit 120 is configured to detect contact between the first terminal 100 and the second terminal 200. Here, the "contact" means physical contact between the first terminal 100 and the second terminal 200. Therefore, even when the first terminal 100 and the second terminal 200 are not electrically connected, the contact detection unit 120 may detect the contact in a case where they are in physical contact with each other. However, the contact detection unit 120 may detect the contact, for example, using a threshold for a degree of contact. For example, the contact detection unit 120 may not detect the contact in a case where a contact area is small between the first terminal 100 and the second terminal 200 and the degree of contact does not exceed the threshold. Conversely, the contact detection unit 120 may detect the contact in a case where the contact area is large between the first terminal 100 and the second terminal 200 and the degree of contact exceeds the threshold.

The contact detection unit 120 may detect the contact using a sensor or similar device provided on at least one of the first terminal 100 and the second terminal 200. However, the method for detecting the contact is not limited to the examples described above. Furthermore, although illustrated here is an example in which the contact detection unit 120 is provided on the first terminal 100, the contact detection unit 120 may be provided on the second terminal 200, instead of the first terminal 100. Alternatively, the contact detection unit 120 may be provided on both the first terminal 100 and the second terminal 200.

The feature quantity transmission unit 130 is configured to transmit the feature quantity acquired by the feature quantity acquisition unit 110, from the first terminal 100 to the second terminal 200. Specifically, the feature quantity transmission unit 130 transmits the feature quantity from the first terminal 100 to the second terminal 200, in a case where the contact between the first terminal 100 and the second terminal 200 is detected by the contact detection unit 120. Therefore, the feature quantity acquired at the first terminal 100 are not transmitted to the second terminal 200 located at a distant position. The feature quantity transmission unit 130 may transmit the feature quantity, for example, using the wireless communication between the first terminal 100 and the second terminal 200, in a case where the contact is detected. The feature quantity transmission unit 130 may also notify the target that the feature quantity 130 is to be transmitted or has been transmitted. For example, the feature quantity transmission unit 130 may audio-output a sound or message for notifying the target that the feature quantity 130 is to be transmitted, from the first terminal 100. Alternatively, the feature quantity transmission unit 130 may display a message or image for notifying the target that the feature quantity 130 is to be transmitted, on a display provided in the second terminal 200.

In a case where the feature quantity acquisition unit 110 has acquired a plurality of feature quantities (e.g., in a case where it stores a plurality of feature quantities acquired in the past), the feature quantity transmission unit 130 may transmit all the plurality of feature quantities to the second terminal 200. Alternatively, the feature quantity transmission unit 130 may transmit only a part of the plurality of feature quantities (e.g., only the most recently acquired one feature quantity) to the second terminal 200. The feature quantity transmission unit 130 may continue transmitting the feature quantity once the contact is detected by the contact detection unit 120, even if the contact is subsequently lost. Alternatively, the feature quantity transmission unit 130 may stop transmitting the feature quantity in a case where the contact is subsequently lost, even though the contact is once detected by the contact detection unit 120.

The ear acoustic authentication unit 210 is configured to perform ear acoustic authentication using the feature quantity transmitted from the feature quantity transmission unit 130. The ear acoustic authentication unit 210 may perform authentication processing on the target, for example, by matching/collating the feature quantity transmitted from the feature quantity transmission unit 130 to/with a feature quantity of a registered user registered in advance. For example, the ear acoustic authentication unit 210 may output a result indicating a success in the authentication (i.e., the target is a registered user), in a case were a matching degree (a degree of match) between the feature quantity transmitted from the feature quantity transmission unit 130 and the feature quantity of the registered user registered in advance exceeds a predetermined authentication threshold. Furthermore, the ear acoustic authentication unit 210 may output a result indicating a failure in the authentication (i.e., the target is not a registered user), in a case were the matching degree between the feature quantity transmitted from the feature quantity transmission unit 130 and the feature quantity of the registered user registered in advance does not exceed the predetermined authentication threshold. The ear acoustic authentication unit 210 may permit a predetermined operation on the second terminal 200, in response to a success in the ear acoustic authentication.

The predetermined operation permitted by the ear acoustic authentication unit 210 may include all the operations that are executable on the second terminal 200, or may be a part of the operations that are executable on the second terminal 200. For example, in a case where the second terminal 200 is configured as a smartphone, the smartphone's lock screen may be unlocked on the condition that the ear acoustic authentication is successful. In this case, the success in the ear acoustic authentication makes it possible to operate the smartphone in general. Alternatively, various smartphone applications may become usable on the condition that the ear acoustic authentication is successful. Furthermore, the predetermined operation on the second terminal 200 may be permitted by another authentication method other than the ear acoustic authentication (e.g., face authentication, fingerprint authentication, passcode authentication, etc.). In this case, the ear acoustic authentication by the ear acoustic authentication unit 210 may skip the other authentication methods and allow the predetermined operation.

Furthermore, the ear acoustic authentication unit 210 may register the feature quantity transmitted from the feature quantity transmission unit 130, as the feature quantity of the registered user. In this case, the ear acoustic authentication unit 210 stores the feature quantity transmitted from the feature quantity transmission unit 130. Subsequently, the ear acoustic authentication unit 210 reads the stored feature quantity when performing the ear acoustic authentication. It then matches a newly acquired feature quantity to the read feature quantity, and performs the authentication processing on the target. The ear acoustic authentication unit 210 may also update the already registered feature quantity of the registered user. In this case, the ear acoustic authentication unit 210 writes and saves the feature quantity transmitted from the feature quantity transmission unit 130, over the already registered feature quantity of the registered user. In a case of registering or updating the feature quantity, the ear acoustic authentication unit 210 may confirm the target whether or not it is permissible to register or update the feature quantity. In this case, the ear acoustic authentication unit 210 may register or update the feature quantity only if the target consents to register or update the feature quantity.

The first terminal 100 may also have the function of the ear acoustic authentication unit 210 described above. In this case, the first terminal 100 may perform ear acoustic authentication, using the feature quantity acquired by the feature quantity acquisition unit 110 as it is. That is, the ear acoustic authentication in the first terminal 100 may be performed without transmitting the feature quantity via the feature quantity transmission unit 130. The ear acoustic authentication in the first terminal 100 may be for the purpose of permitting various operations on the first terminal. For example, in a case where the first terminal 100 is configured as an earphone-type terminal, a success in the ear acoustic authentication in the first terminal 100 may allow music playback in the first terminal 100.

### (Flow of Operation)

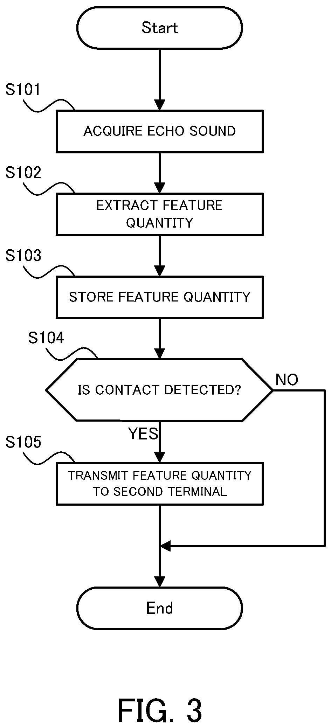

Next, with reference to FIG. 3, a flow of operation of the first information processing system 10 will be described. FIG. 3 is a flowchart illustrating the flow of operation of the first information processing system.

As illustrated in FIG. 3, when the operation of the first information processing system 10 is started, the feature quantity acquisition unit 110 first transmits the sound wave into the target's ear and acquires the echo sound (Step S101). Then, the feature quantity acquisition unit 110 extracts the feature quantity from the acquired echo sound (Step S102). After that, the feature quantity acquisition unit 110 temporarily stores the extracted feature quantity (Step S103).

Then, the contact detection unit 120 detects whether or not the first terminal 100 and the second terminal 200 are in contact (Step S104). In a case where the first terminal 100 and the second terminal 200 are not in contact (Step S104: NO), the subsequent processing is omitted. In this case, the operation may be resumed from the step S101 after a predetermined period.

On the other hand, in a case where the first terminal 100 and the second terminal 200 are in contact (step S104: YES), the feature quantity transmission unit 130 transmits the feature quantity acquired by the feature quantity acquisition unit 110 to the second terminal 200 (step S105). The second terminal 200 may receive the feature quantity transmitted from the feature quantity transmission unit 130 and may perform various types of processing related to the ear acoustic authentication.

### (Technical Effect)

Next, a technical effect obtained by the first information processing system 10 will be described.

As described in FIG. 1 to FIG. 3, in the first information processing system 10, in a case where the contact between the first terminal 100 and the second terminal 200 is detected, the feature quantity used for the ear acoustic authentication is transmitted from the first terminal 110 to the second terminal 200. This enables the appropriate transmission of the feature quantity used for the ear acoustic authentication, from the first terminal 100, which is an acquisition source, to the second terminal 200, which performs the ear acoustic authentication. In a case where the feature quantity acquired at the first terminal 100 is made transmissible even when the contact is not detected, the feature quantity may be transmitted to the second terminal 200 located away from the first terminal 100. Such an operation could potentially lead to theft or leakage of the feature quantity. In the first information processing system 10, however, the feature quantity is transmitted to the second terminal 200 that is in contact with the first terminal 200. That is, since the condition is the contact between terminals, the feature quantity is transmitted and received between terminals located extremely close to each other. Therefore, it is possible to prevent the occurrence of unintended inconveniences such as theft or leakage of the feature quantity.

### <Second Example Embodiment>

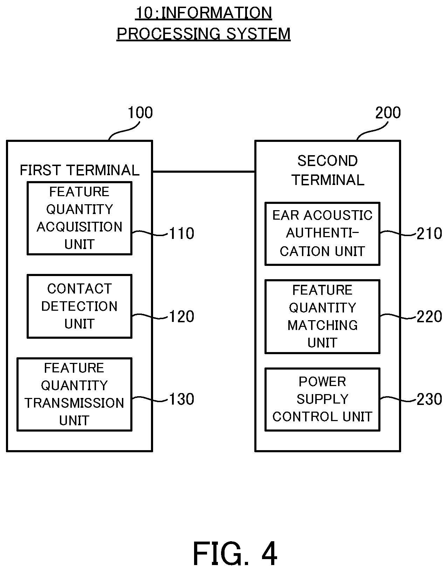

A second information processing system 10 will be described with reference to FIG. 4. Note that the second example embodiment 10 partially differs from the first example embodiment 10 described above only in its operation, and may be the same as the first example embodiment 10 in the other parts. For this reason, a part differing from the first example embodiment will be described in detail below, and a description of the other overlapping parts will be omitted as appropriate.

### (Terminal Configuration)

First, with reference to FIG. 4, a terminal configuration of the second information processing system 10 will be described. FIG. 4 is a block diagram illustrating the terminal configuration of the second information processing system. In FIG. 4, the same components as those illustrated in FIG. 2 carry the same reference numerals.

In FIG. 4, the second information processing system 10 includes the first terminal 100 and the second terminal 200. The first terminal 100 includes, as processing blocks for realizing its functions: the feature quantity acquisition unit 110, the contact detection unit 120, and the feature quantity transmission unit 130. The second terminal 200 includes, as processing blocks for realizing its functions: the ear acoustic authentication unit 210, a feature quantity matching unit 220, and a power supply control unit 230. That is, the second terminal 200 of the second processing apparatus 10 further includes the feature quantity matching unit 220 and the power supply control unit 230, in addition to the configuration described in the first example embodiment (see FIG. 2). Each of the feature quantity matching unit 220 and the power supply control unit 230 may be a processing block configured, for example, by the aforementioned processor 11 (see FIG. 1).

The feature quantity matching unit 220 is configured to perform matching between the feature quantity transmitted by the feature quantity transmission unit 130 and the feature quantity registered in advance in the second terminal 200. The matching here may be similar to the one performed by the ear acoustic authentication unit 210 described above. For example, the feature quantity matching unit 220 may determine whether or not a matching degree (a degree of match) between the feature quantity transmitted from the feature quantity transmission unit 130 and the feature quantity of a registered user registered in advance exceeds the predetermined authentication threshold. In this case, the function of the feature quantity matching unit 220 may be included in the ear acoustic authentication unit 210. For example, the matching performed by the ear acoustic authentication unit 210 (i.e., the ear acoustic authentication) and the matching of feature quantities performed by the feature quantity matching unit 220 may essentially be the same processing.

However, the matching performed by the feature quantity matching unit 220 and the matching performed by the ear acoustic authentication unit 210 may be performed independently and separately. In this case, in the matching performed by the feature quantity matching unit 220 and in the ear acoustic authentication performed by the ear acoustic authentication unit 210, there may be provided different thresholds used for the determination. For example, the threshold in the matching at the feature quantity matching unit 220 may be set to a relatively low value, making it easier to lead to a success than in the ear acoustic authentication performed by the ear acoustic authentication unit 210. Conversely, the threshold in the matching at the feature quantity matching unit 220 may be set to a relatively high value, making it harder to lead to a success than in the ear acoustic authentication performed by the ear acoustic authentication unit 210.

The power supply control unit 230 is configured to control contact power supply between the first terminal 100 and the second terminal 200. Specifically, the power supply control unit 230 may control the initiation of the contact power supply between the first terminal 100 and the second terminal 200 in response to a success in the matching at the feature quantity matching unit 220. Here, the "contact power supply" refers to a power supply method performed without wires or the like (so-called wireless power supply). The method of the contact power supply is not particularly limited, but may be, for example, power supply using electromagnetic induction, magnetic resonance, electric field coupling, microwaves, or the like. A power supply direction in the contact power supply is also not particularly limited. For example, the power supply control unit 230 may perform such control as supplying power from the first terminal 100 to the second terminal 200. Alternatively, the power supply control unit 230 may perform such control as supplying power from the second terminal 200 to the first terminal 100. The power supply direction in the contact power supply may be determined, for example, based on charging states of the first terminal 100 and the second terminal. For instance, the power supply control unit 230 may perform such control as supplying power from a terminal with a higher charge rate to a terminal with a lower charge rate.

### (Flow of Operation)

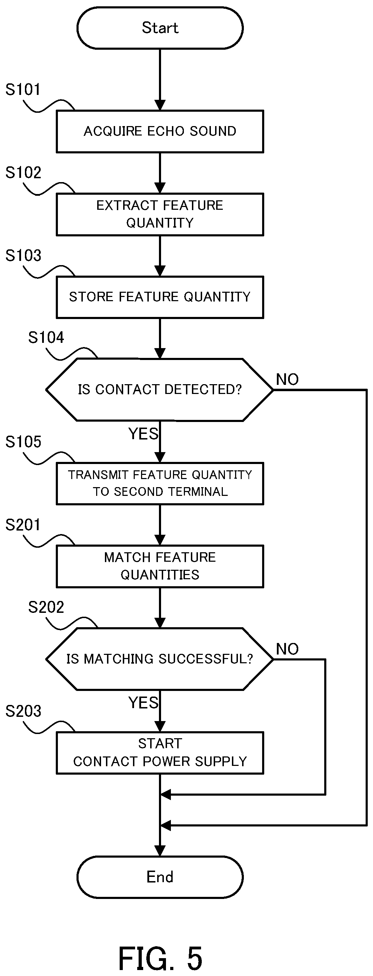

Next, with reference to FIG. 5, a flow of operation of the second information processing system 10 will be described. FIG. 5 is a flowchart illustrating the flow of operation of the second information processing system. In FIG. 5, the same steps as those illustrated in FIG. 3 carry the same reference numerals.

As illustrated in FIG. 5, when the operation of the second information processing system 10 is started, the feature quantity acquisition unit 110 first transmits the sound wave into the target's ear and acquires the echo sound (Step S101). Then, the feature quantity acquisition unit 110 extracts the feature quantity from the acquired echo sound (Step S102). After that, the feature quantity acquisition unit 110 temporarily stores the extracted feature quantity (Step S103).

Then, the contact detection unit 120 detects whether or not the first terminal 100 and the second terminal 200 are in contact (Step S104). In a case where the first terminal 100 and the second terminal 200 are not in contact (Step S104: NO), the subsequent processing is omitted. In this case, the operation may be resumed from the step S101 after a predetermined period.

On the other hand, in a case where the first terminal 100 and the second terminal 200 are in contact (step S104: YES), the feature quantity transmission unit 130 transmits the feature quantity acquired by the feature quantity acquisition unit 110 to the second terminal 200 (step S105). Then, the feature quantity matching unit 220 performs the matching between the feature quantity transmitted by the feature quantity transmission unit 130 and the feature quantity registered in advance in the second terminal 200 (step S201).

In a case where the matching by the feature quantity matching unit 220 is successful (Step S202: YES), the power supply control unit 230 initiates the contact power supply between the first terminal 100 and the second terminal 200 (Step S203). Note that the contact power supply may be terminated in timing in which the charge rate on a power supplying side or a power receiving side reaches a predetermined value. On the other hand, in a case where the matching by the feature quantity matching unit 220 is failed (Step S202: NO), the Step S203 is omitted. That is, the power supply control unit 230 does not initiate the contact power supply between the first terminal 100 and the second terminal 200.

### (Technical Effect)

Next, a technical effect obtained by the second information processing system 10 will be described.

As described in FIG. 4 and FIG. 5, in the second information processing system 10, the contact power supply between the first terminal 100 and the second terminal 200 is initiated in response to a success in the matching of the feature quantity transmitted from the first terminal 100 to the feature quantity registered in the second terminal 200. This prevents unintended contact power supply (e.g., power supply to a terminal held by a person other than the target) from starting. Note that, as described in the first example embodiment, the feature quantity used for the matching is transmitted from the first terminal 100 to the second terminal 200 on the condition that the first terminal 100 and the second terminal 200 are in contact. That is, the feature quantity matching presupposes that the first terminal 100 and the second terminal 200 are in close proximity. Consequently, the contact power supply can be initiated promptly based on a feature quantity matching result.

A processing method that is executed on a computer by recording, on a recording medium, a program for allowing the configuration in each of the example embodiments to be operated so as to realize the functions in each example embodiment, and by reading, as a code, the program recorded on the recording medium, is also included in the scope of each of the example embodiments. That is, a computer-readable recording medium is also included in the range of each of the example embodiments. Not only the recording medium on which the above-described program is recorded, but also the program itself is also included in each example embodiment.

The recording medium to use may be, for example, a floppy disk (registered trademark), a hard disk, an optical disk, a magneto-optical disk, a CD-ROM, a magnetic tape, a nonvolatile memory card, or a ROM. Furthermore, not only the program that is recorded on the recording medium and that executes processing alone, but also the program that operates on an OS and that executes processing in cooperation with the functions of expansion boards and another software, is also included in the scope of each of the example embodiments. In addition, the program itself may be stored in a server, and a part or all of the program may be downloaded from the server to a user terminal. The program may be provided to a user in a form of SaaS (Software as a Service), for example.

### <Supplementary Notes>

The example embodiments described above may be further described as, but not limited to, the following Supplementary Notes below.

### (Supplementary Note 1)

An information processing system according to Supplementary Note 1 is an information processing system including: a first terminal that acquires a feature quantity of an echo sound reflected in a target's ear; a second terminal that enables a predetermined operation based on ear acoustic authentication using the feature quantity; a detection unit that detects contact between the first terminal and the second terminal; and a transmission unit that transmits the feature quantity from the first terminal to the second terminal upon detection of the contact.

### (Supplementary Note 2)

An information processing system according to Supplementary Note 2 is the information processing system according to Supplementary Note 1, further including: a matching unit that performs matching between the feature quantity transmitted by the transmission unit and the feature quantity registered in advance in the second terminal; and a power supply control unit that initiates contact power supply between the first terminal and the second terminal in response to a success in the matching.

### (Supplementary Note 3)

An information processing method according to Supplementary Note 3 is an information processing method that is executed by at least one computer and that controls an information processing system including: a first terminal that acquires a feature quantity of an echo sound reflected in a target's ear; and a second terminal that enables a predetermined operation based on ear acoustic authentication using the feature quantity, the information processing method including: detecting contact between the first terminal and the second terminal; and transmitting the feature quantity from the first terminal to the second terminal upon detection of the contact.

### (Supplementary Note 4)

A recording medium according to Supplementary Note 4 is a recording medium on which a computer program that allows at least one computer to execute an information processing method is recorded, the information processing method being executed by at least one computer and controlling an information processing system including: a first terminal that acquires a feature quantity of an echo sound reflected in a target's ear; and a second terminal that enables a predetermined operation based on ear acoustic authentication using the feature quantity, the information processing method including: detecting contact between the first terminal and the second terminal; and transmitting the feature quantity from the first terminal to the second terminal upon detection of the contact.

### (Supplementary Note 5)

A computer program according to Supplementary Note 5 is a computer program that allows at least one computer to execute an information processing method, the information processing method being executed by at least one computer and controlling an information processing system including: a first terminal that acquires a feature quantity of an echo sound reflected in a target's ear; and a second terminal that enables a predetermined operation based on ear acoustic authentication using the feature quantity, the information processing method including: detecting contact between the first terminal and the second terminal; and transmitting the feature quantity from the first terminal to the second terminal upon detection of the contact.

The present disclosure is allowed to be changed, if desired, without departing from the essence or spirit of this disclosure which can be read from the claims and the entire specification. An information processing system, an information processing method, and a recording medium with such changes are also intended to be within the technical scope of the present disclosure.

### Description of Reference Codes

10 Information processing system
11 Processor
100 First terminal
110 Feature quantity acquisition unit
120 Contact detection unit
130 Feature quantity transmission unit
200 Second terminal
210 Ear acoustic authentication unit
220 Feature quantity matching unit
230 Power supply control unit

## Claims

1. An information processing system comprising:
a first terminal that acquires a feature quantity of an echo sound reflected in a target's ear;
a second terminal that enables a predetermined operation based on ear acoustic authentication using the feature quantity;
a detection unit that detects contact between the first terminal and the second terminal; and
a transmission unit that transmits the feature quantity from the first terminal to the second terminal upon detection of the contact.

2. The information processing system according to Claim 1, further comprising:
a matching unit that performs matching between the feature quantity transmitted by the transmission unit and the feature quantity registered in advance in the second terminal; and
a power supply control unit that initiates contact power supply between the first terminal and the second terminal in response to a success in the matching.

3. An information processing method that is executed by at least one computer and that controls an information processing system including:
a first terminal that acquires a feature quantity of an echo sound reflected in a target's ear; and
a second terminal that enables a predetermined operation based on ear acoustic authentication using the feature quantity,
the information processing method comprising:
detecting contact between the first terminal and the second terminal; and
transmitting the feature quantity from the first terminal to the second terminal upon detection of the contact.

4. A recording medium on which a computer program that allows at least one computer to execute an information processing method is recorded,
the information processing method being executed by at least one computer and controlling an information processing system including:
a first terminal that acquires a feature quantity of an echo sound reflected in a target's ear; and
a second terminal that enables a predetermined operation based on ear acoustic authentication using the feature quantity,
the information processing method comprising:
detecting contact between the first terminal and the second terminal; and
transmitting the feature quantity from the first terminal to the second terminal upon detection of the contact.
